# EUROPÄISCHE PATENTSCHRIFT

(11) **EP 1 305 016 B1**
(45) Veröffentlichungstag und Bekanntmachung des Hinweises auf die Patenterteilung: **02.02.2005**
(21) Anmeldenummer: 01953917.0
(22) Anmeldetag: 14.07.2001
(51) Int. Cl.: A61K 31/16, A61P 17/06

(54) **VERWENDUNG VON N-OLEOYLETHANOLAMIN ZUR BEHANDLUNG DER SCHUPPENFLECHTE (PSORIASIS)**
USE OF N-OLEOYLETHANOLAMINE FOR TREATING PSORIASIS
UTILISATION DE N-OLEOYLETHANOLAMINE POUR TRAITER LE PSORIASIS

(30) Priorität: 29.07.2000 DE 10037046
(43) Veröffentlichungstag der Anmeldung: 02.05.2003
(73) Patentinhaber: Krutmann, Jean, 41844 Wegberg (DE); Grether-Beck, Susanne, 40597 Duesseldorf (DE)
(72) Erfinder: Krutmann, Jean, 41844 Wegberg (DE); Grether-Beck, Susanne, 40597 Duesseldorf (DE)
(74) Vertreter: Miller, Andreas, Dr.
(86) Internationale Anmeldenummer: PCT/DE2001/002630
(87) Internationale Veröffentlichungsnummer: WO 2002/009687

(56) Entgegenhaltungen:
- EP-A- 0 606 590
- WO-A-98/22082
- US-A- 5 851 782
- GRETHER-BECK, S. (1) ET AL: "N-Oleoylethanolamine: A ceramide-degradation inhibitor with antipsoriatic properties." ARCHIVES OF DERMATOLOGICAL RESEARCH, (FEBRUARY, 2001) VOL. 293, NO. 1-2, PP. 37. PRINT. MEETING INFO.: XXVIII ANNUAL MEETING OF THE ARBEITSGEMEINSCHAFT DERMATOLOGISCHE FORSCHUNG IN COOPERATION WITH THE GERMAN DERMATOLOGICAL SOCIETY MUNICH, GERMANY FE, XP001030662

## Beschreibung

Die Erfindung betrifft die Verwendung von N-Oleoylethanolamin zur Herstellung von Arzneimitteln zur Behandlung der Schuppenflechte (Psoriasis) sowie die Behandlung dieser Erkrankung.

N-Oleoylethanolamin [N-(2-Hydroxyethyl)-9(Z)-octadecenamid] der Formel 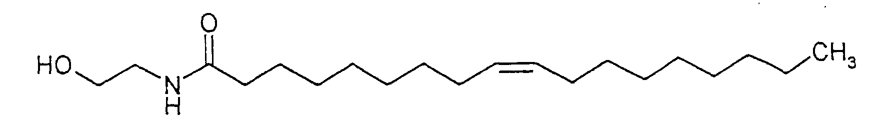 ist eine seit langem bekannte farblose, bei Raumtemperatur feste Substanz, die gut löslich ist in Ethanol. Pharmakologische Effekte wurden erstmals beschrieben von Sugita et al. (Sugita M., Williams M., Dulaney J.T. and Maser H.W. (1975) Ceramidase and ceramide synthesis in human kidney and cerebellum. Biochim. Biophys Acta, 398: 125 - 131). Die Herstellung erfolgt durch Umsetzung von Oleoylchlorid mit Ethanolamin und ist beschrieben von Sugita et al. (Sugita M., Dunlaney J.T, Maser H. W. (1972) Ceramidase deficiency in Farber's disease (lipogranulomatose) Science 178: 100-1102).

N-Oleoylethanolamin fungiert als Strukturanalogon von Ceramiden und vermag die saure Ceramidase zu hemmen mit einem IC₅₀-Wert von 500 µM. In-vitro kommt es dabei zu einer 90%igen Hemmung der sauren Ceramidase. Solche Substanzen wurden damals im Zusammenhang mit der Pathogeneseforschung bei Enzym-Defekt-Erkrankungen gesucht, um die Akkumulation von Ceramiden invivo zu simulieren.

Überraschenderweise wurde jetzt gefunden, dass der Differenzierungsprozess von Keratinozyten durch N-Oleoylethanolamin beinflusst werden kann. Während des Differenzierungsprozesses in den Keratinozyten kommt es zu einer selektiven Wanderung der Zellen von der basalen Schicht in suprabasale Bereiche der Haut (F. M. Watt (1984) Selective migration of terminally differentiating cells from basal layer of cultured human epidermis. J. Cell. Biol. 98: 16 - 21). Im Rahmen dieses Differenzierungsprozesses erfolgt auch eine selektive Genexpression von Differenzierungsmarkern wie Involucrin (F. M. Watt (1983) Involucrin and other markers of keratinocyte terminal differentiation. J. Invest. Dermatol., 81: 100s - 103s) oder Transglutaminase-1. Die vermehrte Expression dieser Gene ist einerseits ein Marker für die Keratinozytendifferenzierung, andererseits hieran kausal beteiligt.

Neben den bekannten Stimuli für die Induktion der Keratinozytendifferenzierung in basalen Keratinozyten in Zellkultur wie etwa Calcium oder Luftkultur konnte zunächst gezeigt werden, dass synthetische Ceramide wie C₂-Ceramid (N-Acetyl-D-erythro-spingosin) oder C₆-Ceramid (N-Hexanoyl-D-erythro-sphingosin) ebenfalls zur Expression der Differenzierungsmarker Involucrin und Transglutaminase-1 führen. Diese genregulatorische Wirkung ist spezifisch, da die Marker für basale Keratinozyten wie Keratin-5 und Keratin-14 nicht induziert werden. In den durchgeführten in-vitro Untersuchungen wurde sodann untersucht, inwieweit die Induktion von den o. g. Differenzierungsmarkern in basalen Keratinozyten statt durch exogene Zugabe der Ceramide C₂ oder C₆ auch durch die Zugabe von Hemmstoffen erfolgen kann, die in der Lage sind, den endogenen Ceramidabbau in den Zellen zu hemmen und damit das Fließgleichgewicht an endogenem Ceramid zu erhöhen.

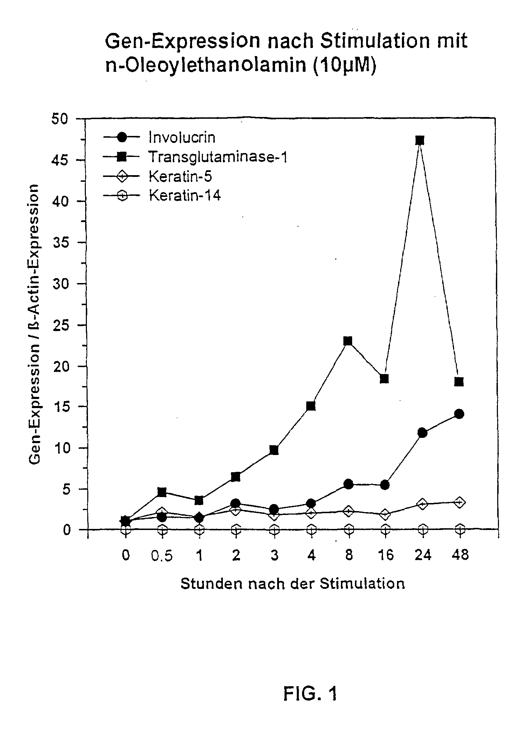

Überraschenderweise wurde gefunden, dass N-Oleoylethanolamin in der Lage ist, ähnlich wie exogen hinzu gegebene Ceramide, die Expression der Differenzierungsmarker Involucrin und Transglutaminase-1 deutlich zu erhöhen. Diese Wirkung ist in Abbildung 1 dargestellt.

Eine Induktion dieser Differenzierungsmarker kann in menschlichen Keratinozyten auch beobachtet werden, wenn sie mit Vitamin D₃ Analoga behandelt werden. Vitamin D₃ Analoga wie z. B. Caiciprotriol oder Tacalzitol werden heute routinemäßig als topisch angewendete Arzneimittel zur Behandlung der Schuppenflechte (Psoriasis) eingesetzt. Ein wesentliches Merkmal der Hautveränderungen bei Psoriasis-Patienten ist eine Störung der Keratinozytendifferenzierung. Genauer gesagt kommt es auf Grund einer überschiessenden Vermehrung basaler Keratinozyten (Hyperproliferation) und einem drastisch erhöhten Turn-Over zu einer Verdickung der Oberhaut (Akanthose), die zudem mit Differenzierungsstörungen der Keratinozyten einhergeht. So kommt es nur zu einer unvollständigen Ausdifferenzierung der Keratinozyten. Es wird heute davon ausgegangen, dass ein wesentlicher Anteil der therapeutischen Wirkung der Vitamin D₃ Analoga auf ihre Fähigkeit zurückzuführen ist, die Differenzierung von Keratinozyten zu induzieren, in dem an der Differenzierung beteiligte Gene wie Transglutaminase-1 und/oder Involucrin induziert werden. Da in den in-vitro Untersuchungen überraschenderweise festgestellt wurde, dass eine Induktion der genannten Differenzierungsmarker auch durch eine Behandlung von kultivierten Keratinozyten mit N-Oleoylethanolamin möglich ist, wurden daraufhin in-vivo Untersuchungen durchgeführt, um zu überprüfen, inwieweit eine topische Behandlung läsionaler Haut von Patienten mit Schuppenflechte mit einem N-Oleoylethanolamin-haltigen Hydrogel zu einer Besserung der Hautveränderungen führen würde.

Im Rahmen eines an zwei Patienten mit Schuppenflechte durchgeführten Heilversuches ergab sich, dass eine Behandlung eines ausgewählten Schuppenflechteherdes mit einem N-Oleoylethanolamin-haltigen Hydrogel, das unter Okklusivbedingungen auf die Haut aufgebracht wurde, zu einer raschen Besserung der klinischen Symptomatik führte. Bei beiden Patienten kam es innerhalb von 3 bzw. 8 Tagen zu einer signifikanten Reduktion der Dicke der psoriatischen Plaques und einem völligen Verlust der Schuppung.

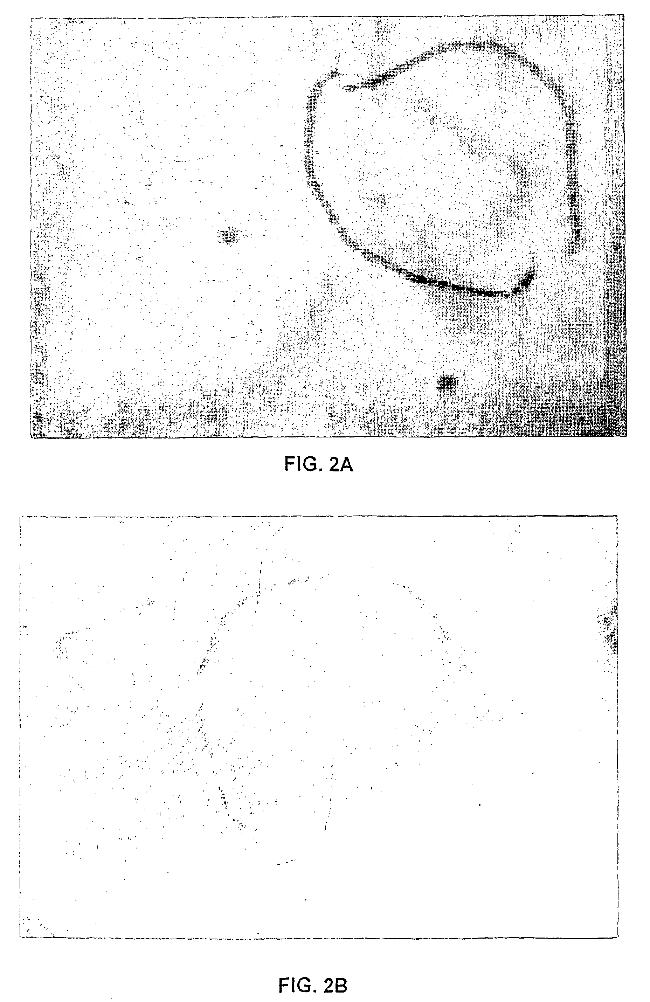

Bemerkenswert ist, dass sich dieser klinisch erkennbare therapeutische Erfolg vergleichsweise rasch einstellte. Während eine Behandlung mit topischen Vitamin D₃ Derivaten in der Regel eine 2 - 3 wöchige Behandlung erfordert, um ein derartiges Ergebnis zu erzielen, konnte dies im Falle des N-Oleoylethanoiaminhaltigen Hydrogels bereits nach wenigen Tagen beobachtet werden. Diese Wirkung ist in Abbildung 2A und 2B dargestellt.

### Beschreibung der Abbildungen:

FIG. 1:
   Gen-Expression der Differenzierungsmarker Involucrin und Transglutaminase-1 in primären humanen Keratinozyten nach einer Gabe von 10µM N-Oleolyethanolamin zwischen 0 und 48 Stunden nach der Stimulation. Die Genexpression wurde als RT-PCR (Mass für die mRNA einer Zelle) bezogen auf die Expression eines Haushaltsgens (hier β-Actin) normiert. Die Basisexpression von unstimulierten Zellen wurde als 1 gesetzt. Die Induktion von Involucrin steigt um den Faktor 15 nach 48 Stunden, während die Induktion von Transglutaminase-1 schon nach 24 Stunden einen Faktor von 48 erreicht. Die Induktion dieser Differenzierungsmarker in den basalen primären Keratinozyten (prolieferierende Zellen, mit geringer Expression der Differenzierungsmarker) ist notwendig für die Differenzierung der Keratinozyten. Als weitere Kontrolle wurde die Expression der basalen Keratine (Strukturelemente in den prolieferierenden Keratinozyten, sie werden bei Differenzierungsprozessen nicht weiter synthetisiert) Keratin-5 und Keratin-14 verfolgt.
FIG. 2A:
   Psoriasisherd, der für vier Tage unbehandelt belassen wurde. Man erkennt neben einer Rötung und Schuppung dieser Hautveränderung vor allen Dingen, dass die Hautveränderung deutlich über das Niveau der Haut hinausragt, d. h. es handelt sich um eine Plaque. Dies ist darauf zurückzuführen, dass die Oberhaut verdickt ist und sich als entzündliches Infiltrat in diesem Hautareal ausgebildet hat.
FIG. 2B:
   Psoriasisherd nach einer viertägigen Behandlung mit einer N-Oleoylethanolamin-haltigen Cremepräparation. Man erkennt hier, dass es zu einer deutlichen Rückbildung der Hautrötung und Schuppung gekommen ist. Besonders bemerkenswert ist jedoch, dass die Hautveränderung sich nach dieser kurzen Behandlungszeit deutlich abgeflacht hat. Es ist nicht mehr zu erkennen und auch nicht mehr zu tasten, dass die Hautveränderung über das Niveau der Haut hinausragt, vielmehr handelt es sich jetzt morphologisch um einen Fleck. Dieser Hautzustand entspricht einem Psoriasisherd, der kurz vor der Abheilung steht.

## Patentansprüche

1. Verwendung von N-Oleoyl-ethanolamin [N-(2-Hydroxyethyl)-9(Z)-octadecenamid] zur Herstellung von Arzneimitteln zur Behandlung der Schuppenflechte (Psoriasis).

2. Verwendung nach Anspruch 1, wobei das Arzneimittel ein N-Oleoyl-ethanolaminhaltiges Hydrogel ist.

3. Verwendung nach Anspruch 1, wobei das Arzneimittel eine N-Oleoyl-ethanolaminhaltige Cremezubereitung ist.

## Claims

1. Use of N-Oleoyl-ethanolamine [N-(2-hydroxyethyl)-9(Z)-octadecenamide] for the manufacturing of pharmaceuticals for the treatment of psoriasis.

2. Use according to claim 1, whereby the pharmaceutical is a N-Oleoyl-ethanolamine in the form of a hydrogel.

3. Use according to claim 1, whereby the pharmaceutical is a N-Oleoyl-ethanolamine in the form of a cream.

## Revendications

1. Utilisation de N-oléoyl-éthanolamine [N-(2-hydroxyéthyl)-9(Z)-octadecenamide] pour la préparation d'un médicament destiné au traitement du psoriasis.

2. Utilisation selon la revendication 1, dans laquelle le médicament est un hydrogel contenant une N-oléoyl-éthanolamine.

3. Utilisation selon la revendication 1, dans laquelle le médicament est une préparation de crème contenant une N-oléoyl-éthanolamine.
